# FASCICULE DE BREVET EUROPEEN

(11) **EP 0 651 881 B1**
(45) Date de publication et mention de la délivrance du brevet: **08.01.1997**
(21) Numéro de dépôt: 93916031.3
(22) Date de dépôt: 22.07.1993
(51) Int. Cl.: G01N 21/89, G01N 33/36, D01G 37/00

(54) **DISPOSITIF DE DETECTION DE DEFAUTS DE MATERIAUX FIBREUX**
VORRICHTUNG ZUR ERFASSUNG VON FEHLERN IN FASERMATERIALIEN
FIBROUS MATERIAL DEFECT DETECTION DEVICE

(30) Priorité: 22.07.1992 FR 9209258
(43) Date de publication de la demande: 10.05.1995
(73) Titulaire: CENTRE DE COOPERATION INTERNATIONALE EN RECHERCHE AGRONOMIQUE POUR LE DEVELOPPEMENT (CIRAD), 75116 Paris (FR)
(72) Inventeur: CORNUEJOLS, Georges, F-34000 Montpellier (FR)
(74) Mandataire: CABINET BONNET-THIRION
(86) Numéro de dépôt international: FR9300753
(87) Numéro de publication internationale: WO9402838

(56) Documents cités:
- EP-A- 0 123 929
- EP-A- 0 196 449
- DE-A- 3 928 279
- GB-A- 1 411 254
- GB-A- 2 095 828
- US-A- 4 648 053

## Description

La présente invention concerne un dispositif de détection et de comptage automatique de défauts de matériaux fibreux.

En particulier, la présente invention s'applique à la mesure du taux de sucres collants dans le coton.

Les matériaux fibreux, généralement d'origine végétale, comme le papier, le coton, le bois, présentent des défauts d'inhomogénéité, des insertions de matériaux n'ayant pas la même maturité, ou des insertions de matériaux d'origine étrangère au procédé d'obtention de ces matériaux: insectes, graines, sucres, sucres collants, déchets artificiels.

Pour détecter ces défauts différents dispositifs ont été mis au point mais ne fonctionnent pas de manière automatique et rapide.

Dans le domaine du coton, les dispositifs connus à ce jour, de type à échauffement et refroidissement d'un voile de carde placé entre deux feuilles d'aluminium, sont limités au comptage oculaire, c'est-à-dire par l'oeil humain, de points de sucres collants sur une feuille d'aluminium.

Le document DE-A-3 928 279 décrit un dispositif dans lequel des corps étrangers particulaires, retenus dans un voile de carde, sont détectés par leur opacité maintenue en contraste avec la translucidité du voile préalablement mouillé.

Le document GB-A-2 095 828 décrit un dispositif où des défauts sont détectés par contraste dans un voile de carde par des capteurs dont l'un est sensible à la lumière réfléchie par le voile de carde et l'autre à la lumière transmise à travers ce voile.

La présente invention prétend remédier à cet inconvénient en présentant un dispositif entièrement automatique permettant de mesurer plusieurs types de défauts de matériaux fibreux, même très fins. En particulier, la présente invention utilise, pour détecter les défauts et les fibres du matériau sur un support artificiel, des polariseurs combinés placés sur la source de lumière et sur un capteur opto-électronique.

Le dispositif objet de la présente invention est un dispositif de détection de défauts, constitués d'éléments à singularité optique, dans des matériaux fibreux, tels notamment que des particules de sucre dans le coton, comportant un capteur opto-électronique capable de fournir des signaux représentatifs de défauts présents dans un champ optique du capteur, qui est relié à un moyen de traitement d'images organisé pour analyser les signaux représentatifs de défauts et les compter, caractérisé en ce qu'il comporte un film de transfert défilant dans le champ du capteur opto-électronique sous l'action d'un moyen de défilement et passant successivement sous un moyen de pressage du matériau fibreux sur le film de transfert, moyen de pressage adapté à faire adhérer à ce dernier les éléments de défaut soit sans fibres, soit liés à des fibres, et sous un moyen de séparation du matériau fibreux d'avec le film, grâce à quoi ce dernier passe dans le champ du capteur en portant les seuls défauts.

Le film de transfert 1 peut être en métal, en matière plastique ou en matériau optiquement absorbant suivant le type de singularité optique des défauts recherchés et la nature des fibres dont on recherche les défauts. Par exemple, il peut être en aluminium pour les défauts de sucre collant dans le coton. Selon un second exemple, le film 1 peut être en papier ou en matériau absorbant pour la détection de défauts de sucre dans le coton. Le film 1 peut, selon un troisième exemple, être teinté, c'est-à-dire absorber partiellement les rayons lumineux pour au moins une longueur d'onde captée par le capteur opto-électronique 6. Le film 1 peut enfin être en matériau selon une combinaison des matériaux selon ces exemples.

Les défauts sans fibres 2 sont liés au film 1 mais ne sont pas liés à des fibres du matériau inspecté. les défauts 3 sont liés au film 1 ainsi qu'à au moins une fibre du matériau inspecté.

Les défauts 2 et 3 se présentent sous forme de taches ou de dépôts sur le film 1. Ils modifient localement l'apparence optique du film 1 telle qu'observée par le capteur opto-électronique 6. Par exemple, les défauts 2 et 3 absorbent partiellement certaines longueurs d'onde ou dépolarisent la lumière ou possèdent un cône de diffusion ou de réflexion différent de celui du film 1.

La source de lumière 5 est adaptée à éclairer les défauts 2 et 3 ainsi que les fibres 4. Par exemple, la source de lumière 5 est constituée d'une ampoule alimentée de manière continue ou de manière synchronisée avec les prises d'images réalisées par le capteur opto-électronique 6 ou d'une source laser balayant le champ optique du capteur opto-électronique 6.

Le capteur opto-électronique 6 est adapté à émettre un signal électrique représentatif de l'image qu'il perçoit. Il est adapté à percevoir une image du film 1, des défauts 2 et 3 et des fibres 4.

Le capteur opto-électronique 6 est, par exemple, constitué d'une caméra à capteur linéaire ou matriciel ou d'une photo-diode.

Le moyen de traitement d'images 7 est adapté à traiter le signal électrique représentatif d'images sortant du capteur opto-électronique 6, à en extraire des informations concernant les défauts 2 et 3 placés dans le champ optique du capteur opto-électronique 6. Le moyen de traitement d'images peut être constitué de circuits électroniques, d'ordinateurs munis de logiciels adaptés ou de réseaux neuronaux, par exemple. Le moyen de traitement d'images 7 peut comporter des connecteurs de sortie des informations résultant des traitements d'images ou une imprimante.

L'afficheur 8 est adapté à afficher les résultats des traitements d'images effectués par le moyen de traitement d'images 7. Cet afficheur peut être par exemple, constitué d'un moniteur d'ordinateur, d'un moniteur vidéo, d'un écran plat.

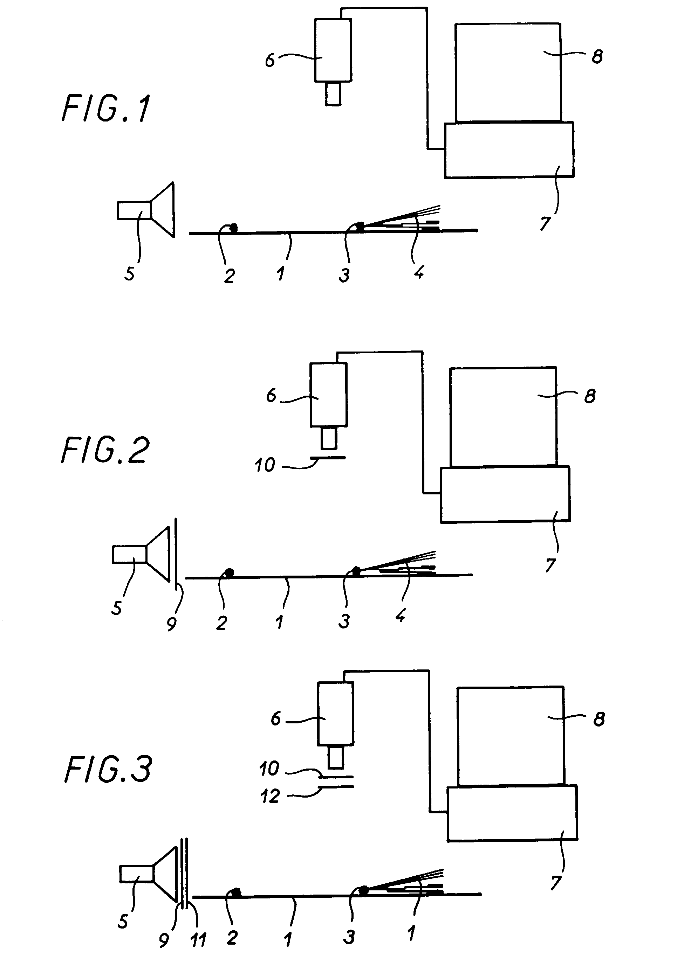

On retrouve dans la figure 2 tous les éléments présentés en figure 1, auxquels s'ajoutent un polariseur 9 lié à la source de lumière 5 et un polariseur 10 lié au capteur opto-électronique 6.

Les polariseurs 9 et 10 sont des polariseurs linéaires, c'est-à-dire que pour au moins une longueur d'onde captée par le capteur opto-électronique 6, ils absorbent les rayons lumineux polarisés selon un plan de polarisation. Les polariseurs 9 et 10 sont croisés, c'est-à-dire que le polariseur 10 est adapté à absorber les rayons lumineux lui parvenant directement du polariseur 9.

La partie optique représentée figure 2 est prévue pour des défauts 2 et 3 et des fibres 4 dépolarisants. Aussi le film 1 est réalisé en matériau non dépolarisant de telle sorte que la lumière polarisée par le polariseur 9 et qui parvient au capteur 6, soit directement, soit réfléchie par le film 1 dans le champ du capteur là où ne sont pas présents des défauts, est arrêtée par le polariseur 10, et le champ apparaît sombre. La lumière partiellement dépolarisée par les défauts 2 et les défauts 3 avec leurs fibres sera admise dans le capteur 6 et apparaîtra en clair sur le fond sombre.

Selon une première variante du mode de réalisation présenté en figure 2, au moins un des polariseurs 9 et 10 est dichroïque, c'est-à-dire qu'il possède deux axes de polarisation orthogonaux polarisant les rayons lumineux de longueurs d'ondes différentes. Selon cette variante, le capteur opto-électronique 6 fonctionne en couleur, c'est-à-dire que le signal électrique représentatif d'images qu'il émet représente aussi la longueur d'onde des rayons lumineux que le capteur opto-électronique perçoit.

Selon une seconde variante du mode de réalisation présenté en figure 2, le polariseur 10 comporte deux zones dont les axes de polarisation sont orthogonaux et le capteur opto-électronique 6 comporte deux zones adaptées à recevoir les rayons lumineux provenant respectivement des deux zones du polariseur. Un tel assemblage peut, par exemple, être réalisé par une caméra comportant deux capteurs séparés par un prisme, les deux faces du prisme placées devant les deux capteurs étant liées à deux polariseurs d'axes de polarisation orthogonaux entre eux.

Selon ces deux variantes, le capteur opto-électronique est adapté à émettre un signal représentatif d'au moins deux images, une de ces images représentant l'apparence optique du film selon une polarisation de lumière, et une autre de ces images représentant l'apparence optique du film selon une seconde polarisation.

On retrouve dans la figure 3 les éléments présentés en figure 2 auxquels s'ajoutent deux lames quart d'onde 11 et 12 placées respectivement entre les polariseurs 9 et 10 et le film 1. On le sait, la combinaison d'un polariseur linéaire et d'une lame quart d'onde donne un polariseur circulaire.

La description de la figure 2 s'applique donc à la figure 3, en remplaçant les considérations sur les polarisations linéaires en considération sur la polarisation circulaire.

Il est à noter que les deux lames quart d'onde fonctionnent avec le même sens de rotation et que la réflexion sur le film 1 provoque un retournement du sens de rotation des rayons lumineux polarisés circulairement. De cette manière, les images captées par le capteur opto-électronique 6 sont claires pour les défauts 2 et 3 ou pour les fibres, respectivement, et sombres pour le film 1.

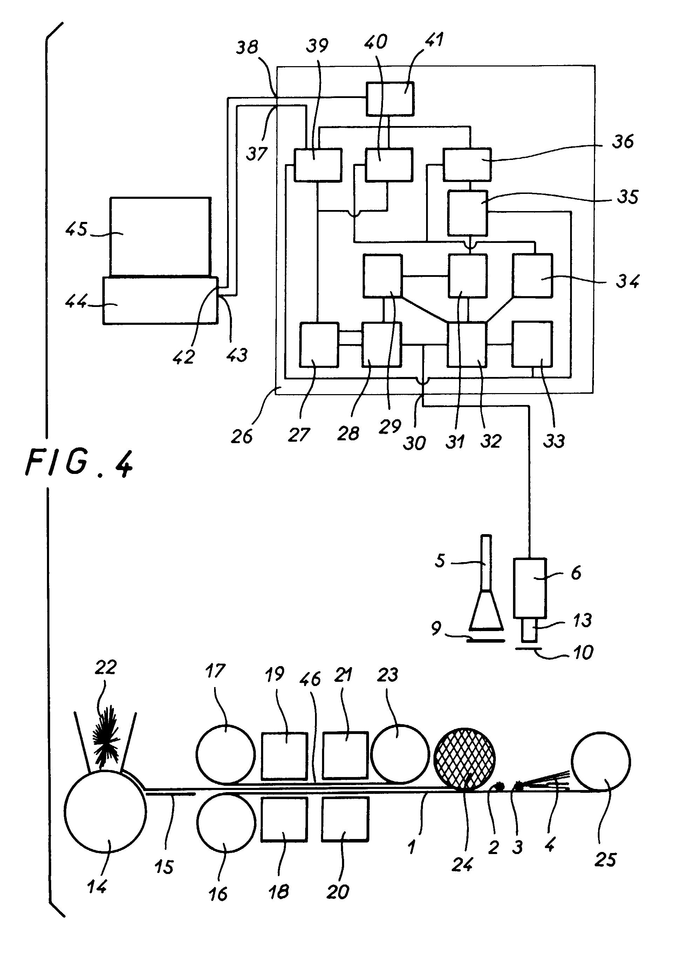

En figure 4 est représenté un dispositif complet adapté à l'inspection de sucre collant dans le coton sur feuilles d'aluminium.

En figure 4 sont représentés le film 1 portant des défauts sans fibre 2 et des défauts 3 liés à des fibres 4, une source de lumière 5 portant un premier polariseur 9, un capteur opto-électronique 6 muni d'un objectif 13 portant un second polariseur 10, un ordinateur 44 comportant une entrée d'informations de défauts 42 et une entrée de signaux de transfert 43, un moniteur 45, un moyen de présentation de fibres en continu 14, un support de voile de fibres 15, un moyen de pressage primaire et distributeur de film 16, un moyen 17 de pressage secondaire et distributeur d'un film supérieur 46, un moyen de chauffage inférieur 18, un moyen de chauffage supérieur 19, un moyen de refroidissement inférieur 20, un moyen de refroidissement supérieur 21, un échantillon de matériau fibreux 22, un moyen de séparation 23 du film supérieur 46, un moyen de séparation du matériau 24, un enrouleur de film inférieur 25, une carte électronique de traitement d'images 26 comportant un compteur 27, un analyseur de synchronisation 28, une mémoire 29, une entrée de carte électronique 30, un comparateur 31, un comparateur à seuil 32, un détecteur de front montant 33, un détecteur de front descendant 34, une porte tampon 35, trois mémoires 36, 39 et 40, un multiplexeur 41, une sortie de signaux de transfert 37, et une sortie d'informations de défauts 38.

La description précise de cette figure est faite dans l'ordre chronologique de transfert de matière et d'informations concernant un échantillon de matériau fibreux 22.

Le déplacement des fibres constituant l'échantillon de matériau fibreux 22 et du film 1 se font de gauche à droite sur la figure 4, un moteur non représenté déplaçant en rotation l'enrouleur 25.

La carte électronique de traitement d'images 26 et l'ordinateur 44 constituent conjointement le moyen de traitement d'images présenté en figures 1 à 3 et 5.

L'échantillon de matériau fibreux 22 est mis en forme par le moyen de présentation de fibres en continu 14, sous la forme d'un voile, appelé voile de carde dans l'industrie cotonnière. Un voile de carde tel que formé par le moyen de présentation en continu 14 est donc formé de fibres provenant successivement de plusieurs échantillons 22. De tels moyens de présentation de fibres en continu sont connus, en particulier dans l'industrie cotonnière.

Le support de voile de fibres 15 est adapté d'une part à séparer le voile du moyen de présentation en continu 14 et d'autre part à supporter en glissement ce voile, vers le moyen de pressage primaire et distributeur de film 16 et vers le moyen de pressage secondaire et distributeur de film 17. Ces deux moyens 16 et 17 se font face, distribuent chacun un film continu, respectivement 1 et 46, constitué d'un matériau de part et d'autre du voile de fibre. Par ailleurs, ces deux moyens 16 et 17 effectuent un pressage des films et du voile intercalé entre ces films. Des moyens de pressage et distributeurs 16 et 17, peuvent, par exemple, être constitués de distributeurs de films d'aluminium ou de films plastiques ou de papiers, dont les axes sont soumis à des forces verticales. Des moyens de pressage et distributeurs 16 et 17, peuvent aussi être constitués de distributeurs de films d'aluminium ou de films plastiques ou de papiers, suivis de rouleaux presseurs de type connu.

Les moyens de chauffage 18 et 19 sont adaptés à vaporiser au moins partiellement l'eau se trouvant dans les fibres et dans les défauts du voile. Les moyens de chauffage peuvent fonctionner par conduction, par exemple en étant constitués de rouleaux chauffés roulant sur les films, par convection forcée, par exemple par soufflage d'air chaud sur au moins un film, et/ou par rayonnement, en étant par exemple constitués d'au moins une plaque plane chauffée et placée à proximité des films.

Il est à noter que les moyens de chauffage peuvent fonctionner de manière asymétrique, le film 1 étant moins chauffé que le film distribué par le moyen de pressage et de distribution 17, de telle manière qu'une condensation s'effectue sur le film 1.

Les moyens de refroidissement 20 et 21 sont adaptés à refroidir les deux films et les fibres intercalées entre les deux films. De tels moyens sont connus.

Le moyen de séparation du film supérieur 23 est adapté à séparer le film supérieur 46 du film 1. Il est, par exemple, constitué d'un enrouleur.

Le moyen de séparation de matériau 24 a pour fonction de séparer le film 1 auquel sont liés les défauts 2 sans fibres, et les défauts 3 avec leurs fibres 4 liées, du voile de carde formé à partir de l'échantillon 22.

Un tel moyen de séparation 24 peut, par exemple, être constitué d'une brosse cylindrique tournant dans le sens des aiguilles d'une montre sur la figure 4 et d'un récupérateur de fibres, non représenté ici. Le moyen de séparation 24 peut aussi être constitué d'un aspirateur.

L'enrouleur de film 25 est adapté à enrouler le film 1 après le passage du film 1 dans le champ de vision du capteur opto-électronique 6.

Le capteur opto-électronique 6 est adapté à émettre un signal électrique représentatif d'images du film 1, des défauts 2 et 3 et des fibres 4 placés entre le moyen de séparation du matériau 24 et l'enrouleur de film 25.

La source de lumière 5 est adaptée à éclairer le champ de vision du capteur opto-électronique 6. Les polariseurs 9 et 10 sont croisés. De cette manière, le signal sortant du capteur opto-électronique 6 est d'amplitude différente pour le film 1, d'une part et pour les défauts 2 et 3 et les fibres 4, d'autre part.

Les signaux représentatifs d'images sortant du capteur opto-électronique 6 rentrent dans la carte électronique de traitement d'images 26 par son entrée 30 et parviennent à l'analyseur de synchronisation 28, d'une part et au comparateur à seuil 32, d'autre part.

L'analyseur de synchronisation est de type connu. Il fournit au compteur 27 d'une part un signal de changement de ligne correspondant à la fin, dans le signal émis par le capteur opto-électronique 6, des informations concernant une ligne de son champ optique, et d'autre part un signal de changement de points sur la ligne. Le compteur 27 est adapté à compter les signaux de changement de points et est remis à zéro par le signal de changement de ligne. Il fournit aux mémoires 39 et 40 le numéro du point de la ligne en cours de sortie du capteur opto-électronique 6. Le comparateur à seuil 32 est adapté à émettre un signal de valeur logique "1" pour les signaux représentatifs d'images correspondant aux défauts 2 et 3 et aux fibres 4 et un signal de valeur logique "0" pour les signaux représentatifs d'images correspondant au film 1.

La mémoire 29 est adaptée à conserver pendant la durée de sortie d'informations du capteur opto-électronique 6 correspondant à une ligne complète de son champ optique, à recevoir les informations sortant du comparateur à seuil 32 et à émettre les informations qu'elle a conservées vers le comparateur 31.

Le comparateur 31 compare les valeurs logiques sortant du comparateur à seuil 32, d'une part et de la mémoire 29, d'autre part. Le signal logique sortant du comparateur 31 est "1" lorsqu'il reçoit deux signaux de valeurs logiques "1" et "0" dans tous les autres cas. Le comparateur 31 peut, par exemple, être constitué d'une porte "ET". Il constitue ainsi un moyen de détection de continuité de défauts entre deux signaux de ligne successivement émis par le capteur opto-électronique.

La porte tampon 35 est adaptée à conserver la valeur logique la plus élevée sortant du comparateur 31 entre deux remises à zéro. Elle peut donc être constituée d'une bascule, par exemple.

Le détecteur de front montant 33 est adapté à émettre un signal de valeur logique "1" lors du passage, en sortie du comparateur à seuil 32 d'une valeur logique "0" à une valeur logique "1".

La sortie du détecteur de front montant 33 est reliée d'une part à l'entrée de remise à zéro de la porte tampon 35 et d'autre part à l'entrée d'ordre d'écriture de la mémoire 39.

Le détecteur de front descendant 34 est adapté à émettre un signal de valeur, logique "1" lors du passage, en sortie du comparateur à seuil 32 d'une valeur logique "1" à une valeur logique "0".

La sortie du détecteur de front descendant 34 est reliée aux entrées d'ordre d'écriture dans les mémoires 36 et 40.

Selon ce schéma électronique, pour chaque défaut 2 ou 3 et pour chaque fibre 4 apparaissant sur une ligne d'inspection du capteur opto-électronique 6 sur le film 1, la mémoire 36 conserve l'information de continuité de défauts entre les deux signaux de ligne successifs précédemment émis par le capteur opto-électronique 6, la mémoire 40 conserve le dernier numéro du point de la ligne correspondant audit défaut ou fibre, et la mémoire 39 conserve le premier numéro de point de la ligne correspondant audit défaut ou fibre.

Les mémoires 36, 39 et 40 peuvent, par exemple, être constituées de mémoires dites "premier entré, premier sorti", plus connues sous leur nom anglais de FIFO, initiales des mots anglais "First In, First Out". La mémoire 39 est adaptée à émettre un signal de remplissage partiel ou total sur la sortie de signaux de transfert 37. Lorsqu'il reçoit ce signal, l'ordinateur 44 effectue une lecture des mémoires 36, 39 et 40, par l'intermédiaire du multiplexeur 41, selon des procédés et moyens connus.

Le traitement des informations reçues par l'ordinateur 44 sur son entrée d'informations de défauts 42 est simple.

Pour compter le nombre total de défauts apparaissant pendant une durée donnée, par exemple 20 secondes selon la norme de mesure de paramètres du coton connue sous le nom de HVI, le moyen de traitement 1 additionne la valeur "1" à une valeur en mémoire pour chaque valeur logique "0" provenant de la mémoire 36.

Pour séparer les défauts 2 des défauts 3 et des fibres 4, l'ordinateur 44 sélectionne les défauts et les fibres liées à ces défauts en fonction d'au moins une de leurs dimensions, par exemple leur longueur. A cet effet, il comporte un comparateur à seuil d'au moins une dimension des défauts. De cette manière, les défauts ou points de sucre 2 non liés à des fibres et apparaissant donc avec une longueur très faible sont séparés des défauts ou sucres collants 3 liés à des fibres 4 et apparaissant avec une longueur importante.

Il est à noter que le mode de réalisation de la partie optique du dispositif selon l'invention présenté en figure 4 peut être remplacé par l'un quelconque des modes de réalisation présentés en figures 1 à 3 ou 5.

Il est à noter que le comparateur à seuil 32 peut comporter plusieurs seuils adaptés à la détection de plusieurs types de défauts différents.

Il est à noter qu'une carte électronique telle que la carte électronique de traitement d'images 26 présentée en figure 4 n'est pas indispensable au fonctionnement du dispositif, une numérisation dans une mémoire d'ordinateur et un logiciel de traitement d'images pouvant effectuer les mêmes fonctions ou d'autres fonctions donnant des résultats identiques. La carte électronique de traitement d'images 26 est présentée à titre d'exemple de traitements d'images rapides pouvant être utilisés pour le fonctionnement du dispositif selon l'invention.

Selon une variante du moyen de traitement d'images 7, la carte électronique de traitement d'images 26 où l'ordinateur 44 comporte un moyen de mesure du nombre de points photosensibles d'une même ligne du capteur opto-électronique percevant des rayons lumineux provenant du même défaut.

Selon une première variante du dispositif présenté en figure 4, le film 1 est adapté à absorber au moins partiellement certains liquides et les défauts présentent, en au moins un endroit entre le moyen de pressage et le moyen de séparation une phase liquide, cette phase liquide pouvant être générée par le chauffage du film 1 réalisé par les moyens de chauffage 18 et 19.

Selon une seconde variante du dispositif présenté en figure 4, les moyens de chauffage sont incorporés aux moyens de pressage, les films 1 et 46 étant chauffés avant d'être pressés sur le voile de fibres provenant de l'échantillon fibreux 22.

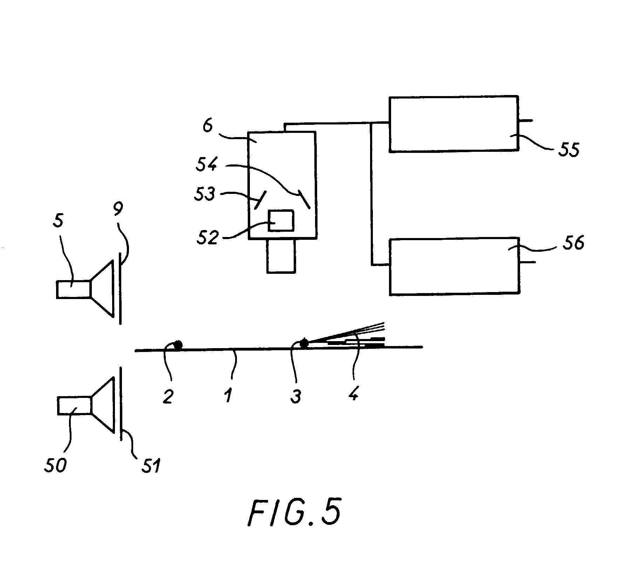

Dans la figure 5 sont représentés le film 1, les défauts sans fibres 2, les défauts 3 liés à des fibres 4, deux sources de lumière 5 et 50 placées de part et d'autre du film 1 et portant deux polariseurs 9 et 51, respectivement, un capteur optique 6 comportant un séparateur de polarisations 52, deux zones de capteurs 53 et 54, un additionneur de signaux 55 et un soustracteur de signaux 56.

Le film 1 est ici constitué d'un matériau partiellement transmissif, comme par exemple du papier, du buvard, du tissu.

Les polariseurs 9 et 51 sont croisés entre eux. Les sources de lumière 5 et 50 sont adaptées à émettre une lumière égale et uniforme sur les deux faces du film 1.

Les deux sources de lumière 5 et 50 sont placées de part et d'autre du film et adaptées à émettre, pour les points du film 1 ne comportant pas de défaut, des lumières transmises en direction du capteur opto-électronique sensiblement égales.

Le séparateur de polarisation 52 est de type connu et est adapté à séparer les rayons lumineux en fonction de leur polarisation, à envoyer à la zone de capteur 53 les rayons lumineux polarisés parallèlement à l'axe de polarisation du polariseur 9 et à envoyer à la zone de capteur 54 les rayons lumineux polarisés parallèlement à l'axe de polarisation du polariseur 51.

Les deux zones de capteur 53 et 54 peuvent être constituées de deux capteurs d'images électroniques, par exemple. Elles captent des images de manière synchronisée et émettent des signaux synchronisés, point par point, de telle manière que les informations d'images sortant des deux zones de capteur et correspondant au même point du film 1 sortent simultanément des deux zones de capteur.

Le capteur opto-électronique 6 est ainsi adapté à émettre un signal représentatif d'au moins deux images, une de ces images représentant l'apparence optique du film selon un axe de polarisation de lumière et une autre de ces images représentant l'apparence optique du film selon un second axe de polarisation.

L'additionneur de signaux 55 est adapté à additionner les signaux sortant des deux zones de capteur 53 et 54.

Le soustracteur 56 est adapté à soustraire les deux signaux sortant des deux zones de capteur 53 et 54.

Selon le type de défaut recherché, le moyen de traitement d'images 7 est relié soit à la sortie de l'additionneur de signaux 55, soit à la sortie du soustracteur de signaux 56.

Il est à noter que selon une variante du mode de réalisation présenté en figure 5, les polariseurs 9 et 51 sont supprimés et le capteur opto-électronique 6 est tel que présenté en figure 1.

## Revendications

1. Dispositif de détection de défauts, constitués d'éléments à singularité optique, dans des matériaux fibreux, tels notamment que des particules de sucre dans le coton, comportant un capteur opto-électronique (6) capable de fournir des signaux représentatifs de défauts présents dans un champ optique du capteur (6), qui est relié à un moyen de traitement d'images (7, 26, 44) organisé pour analyser les signaux représentatifs de défauts et les compter, caractérisé en ce qu'il comporte un film (1) de transfert défilant dans le champ du capteur opto-électronique sous l'action d'un moyen de défilement (25) et passant successivement sous un moyen de pressage (16, 17) du matériau fibreux (22) sur le film de transfert (1), moyen de pressage (16, 17) adapté à faire adhérer à ce dernier les éléments de défaut soit sans fibres (2), soit liés (3) à des fibres (4), et sous un moyen de séparation (24) du matériau fibreux (22) d'avec le film (1), grâce à quoi ce dernier passe dans le champ du capteur (6) en portant les seuls défauts (2, 3, 4).

2. Dispositif selon la revendication 1, pour des défauts (2, 3, 4) dispersifs pour au moins une longueur d'onde de lumière polarisée, caractérisé en ce que, le film (1) étant choisi en un matériau non dispersif pour ladite longueur d'onde de lumière polarisée, le dispositif comporte une source de lumière (5) munie d'un premier polariseur (9) filtrant la lumière émise par la source pour délivrer sur le film de transfert (1) dans le champ du capteur (6) ladite lumière polarisée, et un second polariseur (10) disposé sur le capteur opto-électronique (6) et orienté pour arrêter ladite lumière polarisée non dispersée.

3. Dispositif selon la revendication 1 ou la revendication 2, caractérisé en ce que, sur le trajet de défilement du film (1) entre le moyen de pressage (16, 17) et le moyen de séparation (24) est disposé un moyen de chauffage (18, 19) qui chauffe le film (1) pour y faire adhérer les défauts (2, 3, 4).

4. Dispositif selon l'une des revendications 1 à 3, où le capteur opto-électronique (6) émet une succession de signaux représentatifs de lignes transversales du film (1) comportant un nombre déterminé de points, caractérisé en ce que le moyen de traitement d'images (26) comporte un moyen (31) pour détecter une continuité de défauts (2, 3, 4) entre signaux représentatifs de lignes successifs.

5. Dispositif selon la revendication 4, caractérisé en ce que le moyen de traitement d'images (26) comporte des moyens (27, 39, 40) de comptage du nombre de points d'une ligne affectés par un même défaut (2, 3, 4).

6. Dispositif selon l'une des revendications 4 et 5, caractérisé en ce qu'il comporte un comparateur à seuil propre à sélectionner des défauts (2, 3, 4) en fonction d'au moins une de leurs dimensions.

7. Dispositif selon la revendication 1, caractérisé en ce qu'il comporte deux sources lumineuses (5, 50) disposées de part et d'autre du film de transfert (1) et telles que les lumières qu'elles émettent, transmises par le film (1) dans le champ capteur opto-électronique (6) soient sensiblement égales, en l'absence de défauts (2, 3, 4).

8. Dispositif selon la revendication 7, caractérisé en ce que les deux sources lumineuses (5, 50) sont munies chacune d'un polariseur respectif (9, 51) disposé avec leurs axes de polarisation en quadrature, le capteur opto-électronique (6) étant équipé d'un séparateur de lumière polarisée (52) et de deux zones photosensibles (53, 54), le séparateur (52) étant disposé en sorte de transmettre aux deux zones (53, 54) la lumière polarisée parallèlement aux axes des polariseurs respectifs (9) et (51).

9. Dispositif selon la revendication 1, caractérisé en ce que le film de transfert (1) est choisi pour absorber partiellement la lumière à au moins une longueur d'onde, tandis que le capteur opto-électronique (6) est sensible, sélectivement, à cette longueur d'onde.

## Patentansprüche

1. Vorrichtung zum Auffinden von Fehlern, die aus Teilchen mit optischer Eigentümlichkeit bestehen, in faserigen Materialien, wie insbesondere von Zuckerpartikeln in der Baumwolle, umfassend ein opto-elektronisches Aufnahmegerät (6), das für die in einem Sichtfeld des Aufnahmegerätes (6) vorhandenen Fehler repräsentative Signale liefern kann und das mit einem Mittel zur Bildverarbeitung (7, 26, 44) verbunden ist, das für das Analysieren der für die Fehler repräsentativen Signale und ihre Zählung eingerichtet ist, dadurch gekennzeichnet, daß sie einen Transportfilm (1) umfaßt, der unter der Einwirkung eines Mittels zum Abspulen (25) durch das Feld des opto-elektronischen Aufnahmegerätes läuft und nacheinander unter einem Mittel zum Pressen (16, 17) des faserigen Materials (22) auf den Transportfilm (1), wobei das Mittel zum Pressen (16, 17) so eingerichtet ist, daß die fehlerhaften Teilchen, sei es ohne Fasern (2), sei es verbunden (3) mit Fasern (4), an diesem letzteren anhaften können, und unter einem Mittel zum Abtrennen (24) des faserigen Materials (22) vom Film (1) entlangläuft, aufgrund dessen dieser letztere durch das Feld des Aufnahmegerätes (6) läuft, während er die einzelnen bzw. vereinzelten Defekte (2, 3, 4) trägt.

2. Vorrichtung nach Anspruch 1 für Fehler (2, 3, 4), die für mindestens eine Wellenlänge polarisierten Lichtes lichtstreuend sind, dadurch gekennzeichnet, daß, während der Film (1) unter einem Material ausgewählt ist, das für die genannte Wellenlänge polarisierten Lichtes nicht lichtstreuend ist, die Vorrichtung eine Lichtquelle (5), die mit einem ersten Polarisator (9) versehen ist, der das von der Quelle ausgesandte Licht filtriert, um auf dem Transportfilm (1) im Feld des Aufnahmegerätes (6) das genannte polarisierte Licht bereitzustellen, und einen zweiten Polarisator (10) umfaßt, der auf dem opto-elektronischen Aufnahmegerät (6) angeordnet und so ausgerichtet ist, daß er das genannte polarisierte, nicht gestreute Licht abfängt.

3. Vorrichtung nach Anspruch 1 oder Anspruch 2,
dadurch gekennzeichnet,
daß auf dem Weg des Entlanglaufens des Films (1) zwischen dem Mittel zum Pressen (16, 17) und dem Mittel zum Abtrennen (24) ein Mittel zum Aufheizen (18, 19) angeordnet ist, das den Film (1) erwärmt, damit die Fehler (2, 3, 4) darauf anhaften können.

4. Vorrichtung nach einem der Ansprüche 1 bis 3, worin das opto-elektronische Aufnahmegerät (6) eine Abfolge von für transversale Abschnitte des Films (1) repräsentativen Signalen emittiert, die eine vorgegebene Anzahl von Punkten tragen, dadurch gekennzeichnet,
daß das Mittel zum Bildverarbeiten (26) ein Mittel (31) zum Auffinden einer Abfolge von Fehlern (2, 3, 4) zwischen für aufeinanderfolgende Abschnitte repräsentativen Signalen umfaßt.

5. Vorrichtung nach Anspruch 4, dadurch gekennzeichnet, daß das Mittel zum Bildverarbeiten (26) Mittel (27, 39, 40) zum Zählen der Anzahl von Punkten eines Abschnitts umfaßt, die durch ein und denselben Fehler (2, 3, 4) beeinflußt sind.

6. Vorrichtung nach einem der Ansprüche 4 und 5,
dadurch gekennzeichnet,
daß sie einen Schwellenkomparator umfaßt, der geeignet ist, Fehler (2, 3, 4) in Abhängigkeit von mindestens einer ihrer Abmessungen auszuwählen.

7. Vorrichtung nach Anspruch 1, dadurch gekennzeichnet, daß sie zwei Lichtquellen (5, 50) umfaßt, die beiderseits des Transportfilms (1) angeordnet sind und zwar so, daß in der Abwesenheit von Fehlern (2, 3, 4) das Licht, das sie emittieren und das durch bzw. über den Film (1) in das Feld des opto-elektronischen Aufnahmegerätes (6) fällt, spürbar gleichförmig ist.

8. Vorrichtung nach Anspruch 7, dadurch gekennzeichnet, daß die beiden Lichtquellen (5, 50) jeweils mit einem entsprechenden Polarisator (9, 51) versehen sind, der zu ihren Polarisationsachsen um 90° versetzt angeordnet ist, wobei das opto-elektronische Aufnahmegerät (6) mit einem Separator für polarisiertes Licht (52) und zwei photoempfindlichen Zonen (53, 54) ausgestattet ist, wobei der Separator (52) so angeordnet ist, daß er das Licht, das parallel zu den Achsen der jeweiligen Polarisatoren (9) und (51) polarisiert ist, zu den beiden Zonen (53, 54) durchfallen läßt.

9. Vorrichtung nach Anspruch 1, dadurch gekennzeichnet, daß der Transportfilm (1) so ausgewählt ist, daß er das Licht mindestens einer Wellenlänge teilweise absorbiert, während das opto-elektronische Aufnahmegerät (6) selektiv für diese Wellenlänge empfindlich ist.

## Claims

1. A device for detecting defects, comprising elements which are optically distinctive, in fibrous materials, in particular such as sugar particles in cotton, comprising an optoelectronic sensor (6) able to supply signals which are representative of defects present in an optical field of the sensor (6), which is connected to an image-processing means (7,26,44) designed to analyse the signals which are representative of defects and to count them, characterised in that it comprises a transfer film (1) passing in the field of the optoelectronic sensor under the action of a transport means (25) and passing successively under a means (16,17) for pressing fibrous material (22) on to the transfer film (1) adapted to cause to adhere to the latter the defective elements either without fibres (2) or attached (3) to fibres (4), and under a means (24) for separating the fibrous material (22) from the film (1), as a result of which the latter enters the field of the sensor (6) carrying therewith the individual defects (2,3,4).

2. A device according to claim 1, for defects (2,3,4) which are dispersive for at least one wavelength of polarised light, characterised in that, with the film (1) being chosen from a material which is not dispersive for said wavelength of polarised light, the device comprises a light source (5) provided with a first polariser (9) filtering the light emitted by the source so as to deliver said polarised light on to the transfer film (1) in the field of the sensor (6), and a second polariser (10) disposed on the optoelectronic sensor (6) and orientated so as to arrest said non-dispersed polarised light.

3. A device according to claim 1 or claim 2, characterised in that in the transport path of the film (1) between the pressing means (16,17) and the separating means (24) there is provided a heating means (18,19) which heats the film (1) so as to cause the defects (2,3,4) to adhere to it.

4. A device according to any one of claims 1 to 3, in which the optoelectronic sensor (6) emits a series of signals which are representative of transverse lines on the film (1) comprising a given number of points, characterised in that the image-processing means (26) comprises a means (31) for detecting a continuity of defects (2,3,4) between signals representative of successive lines.

5. A device according to claim 4, characterised in that the image-processing means (26) comprises means (27,39,40) for counting the number of points of a line affected by the same defect (2,3,4).

6. A device according to either one of claims 4 and 5, characterised in that it comprises a comparator with a threshold adapted to select the defects (2,3,4) as a function of at least one of their dimensions.

7. A device according to claim 1, characterised in that it comprises two light sources (5,50) which are disposed on either side of the transfer film (1) and which are such that the amount of light which they emit, transmitted through the film (1) in the field of the optoelectronic sensor (6), is substantially equal in the absence of defects (2,3,4).

8. A device according to claim 7, characterised in that the two light sources (5,50) are each provided with a respective polariser (9,51), which are arranged with their polarisation axes in quadrature, the optoelectronic sensor (6) being provided with a separator (52) for polarised light and two photosensitive zones (53,54), the separator (52) being arranged so as to transmit to the two zones (53,54) the polarised light parallel to the axes of the respective polarisers (9) and (51).

9. A device according to claim 1, characterised in that the transfer film (1) is chosen so as to absorb partially the light at at least one wavelength, whereas the optoelectronic sensor (6) is selectively sensitive at this wavelength.
